(19) Europäisches Patentamt
European Patent Office
Office européen des brevets

(11) Numéro de publication: **0 092 450**
**B1**

# (12) FASCICULE DE BREVET EUROPEEN

(45) Date de publication du fascicule du brevet: **03.06.87**

(21) Numéro de dépôt: **83400555.5**

(22) Date de dépôt: **17.03.83**

(51) Int. Cl.$^4$: **C 07 D 209/16,**
C 07 D 209/42, A 61 K 31/40

## (54) Nouveaux dérivés de la tryptamine actifs notamment sur le système cardiovasculaire et procédé pour leur préparation.

(30) Priorité: **23.03.82 FR 8204921**

(43) Date de publication de la demande:
**26.10.83 Bulletin 83/43**

(45) Mention de la délivrance du brevet:
**03.06.87 Bulletin 87/23**

(84) Etats contractants désignés:
**AT BE CH DE FR GB IT LI LU NL SE**

(56) Documents cités:
**EP-A-0 025 111**
**EP-A-0 025 727**
**EP-A-0 045 911**
**EP-B-0 014 951**
**GB-A-2 001 633**

**Le dossier contient des informations techniques présentées postérieurement au dépôt de la demande et ne figurant pas dans le présent fascicule.**

(73) Titulaire: **SANOFI, société anonyme**
**40, Avenue George V**
**F-75008 Paris (FR)**

(72) Inventeur: **Wagnon, Jean**
**195 rue Michel Ange**
**F-34000 Montpellier (FR)**
Inventeur: **Gautier, Patrick**
**Manavieille**
**F-34660 Cournonterral (FR)**
Inventeur: **Gagnol, Jean-Pierre**
**Place de l'Eglise**
**F-34380 St. Martin de Londres (FR)**

(74) Mandataire: **Gillard, Marie-Louise et al**
**Cabinet Beau de Loménie 55, Rue d'Amsterdam**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 092 450 B1

Courier Press, Leamington Spa, England.

**Description**

Les demandes de brevet européen publiées sous les numéros 14 951 et 45 911 décrivent des composés à action β-bloquante dérivés de l'hydroxy-4 indole.

La demande de brevet anglais publiée sous le n° 2 001 633 décrit des dérivés de la diméthyltryptamine substitués sur l'azote aliphatique par un groupement aryloxy-3 hydroxy-2 propyle.

Enfin la demande de brevet européen n° 25 727 décrit des dérivés de la méthyltryptamine substitués sur l'azote aliphatique par un groupe aryloxy ou indolyloxy-3 hydroxy-2 propyle.

L'objet de la présente invention est de fournir des composés doués d'activité β-bloquante et dépourvus autant que possible des activités secondaires parfois gênantes rencontrées dans ce type de produits lors du traitement de l'hypertension.

La présente invention concerne en tant que produits nouveaux des substances chimiques dérivées de la tryptamine ainsi que leurs sels d'addition d'acides.

L'invention concerne également un procédé de préparation de ces composés ainsi que leur application en thérapeutique.

Les composés selon l'invention sont choisis parmi l'ensemble constitué par:

a) Les composés répondant à la formule générale:

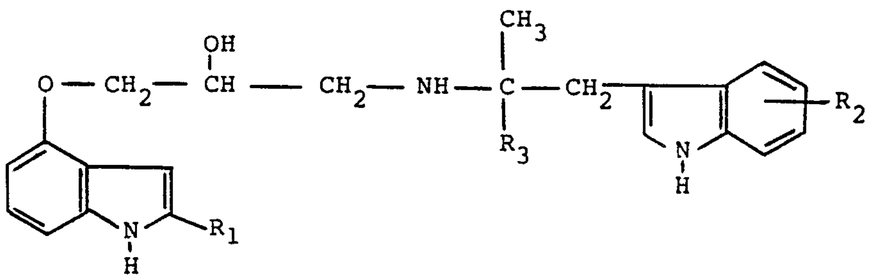

(I)

dans laquelle

$R_1$ représente un groupe $CH_2OH$, un groupe —C≡N, un groupe

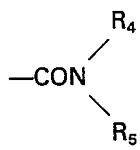

ou encore un groupe $—COOR_6$ dans lesquels $R_4$, $R_5$ et $R_6$ désignent un groupe alkyle inférieur ou l'hydrogène;

$R_2$ représente l'hydrogène ou un groupe C≡N;

$R_3$ désigne l'hydrogène, un groupe $CH_3$ ou encore un groupe $CH_2OH$.

b) Les sels d'addition que les composés (I) sont susceptibles de donner avec les acides minéraux ou organiques pharmaceutiquement acceptables tels que l'acide chlorhydrique, l'acide citrique, l'acide maléique, l'acide fumarique, l'acide tartrique et l'acide acétique.

Dans le présent brevet, on entend par groupe alkyle inférieur un groupe alkyle droit ou ramifié comportant de 1 à 4 atomes de carbone.

Lorsque $R_3$ représente un groupe $CH_3$, les composés (I) ne comportent qu'un seul atome de carbone asymétrique au niveau de la fonction alcool et peuvent exister sous forme de deux isomères optiques R et S. Lorsque $R_3$ est H ou $CH_2OH$, le carbone portant $R_3$ est lui-même un atome de carbone asymétrique et les composés (I) possèdent deux centres d'asymétrie. Par suite, il existe quatre stéréoisomères des composés (I): RR, RS, SR et SS. Aussi bien les isomères optiques que les stéréoisomères font partie intégrante de l'invention.

Les composés (I) peuvent être obtenus par la réaction suivante:

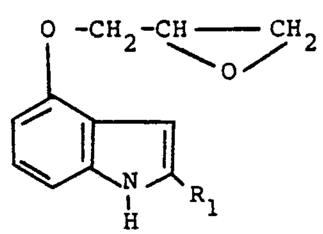

+

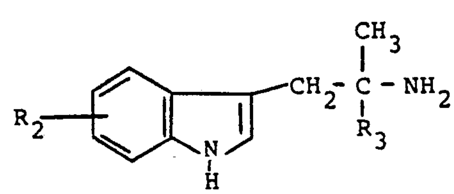

2

1

La condensation des époxydes *1* avec les amines *2* peut s'effectuer par chauffage entre 100 et 140°C des deux substances en quantités sensiblement équimoléculaires. Lorsque la vitesse de réaction est trop lente, on peut l'accélérer par addition d'un catalyseur tel que le phénol.

La condensation peut également être réalisée par chauffage des deux réactifs en solution dans un solvant hydroxylé tel que l'éthanol. Le plus souvent on opère à la température d'ébullition du solvant.

En ce qui concerne les produits de départ:

— Les époxydes *1* sont connus. On peut en particulier les obtenir par action de l'épichlorhydrine sur le phénol correspondant. Dans le cas particulier où $R_1$ représente —C≡N, l'époxyde *1* s'obtient de façon connue par déshydratation de l'époxyde où $R_1$ est —$CONH_2$.

— Les dérivés de la tryptamine *2* sont connus ou peuvent être obtenus par des procédés connus. En particulier lorsque $R_3$ désigne H ou $CH_3$, les composés *2* peuvent être préparés à partir des dérivés de la gramine selon la méthode décrite dans Journal of American Chemical Society *69,* 3140—3142, (1947).

Dans le cas où $R_3$ désigne —$CH_2OH$, les composés *2* peuvent être obtenus par réduction des dérivés correspondants du tryptophane selon la méthode décrite dans Biochemica Biophysica Acta, *341,* 284, (1974).

Enfin, dans le cas où $R_2$ désigne C≡N, les composés 2 peuvent être obtenus à partir du dérivé bromé correspondant par action du cyanure cuivreux au sein d'un solvant tel que la N-méthylpyrrolidone.

Les exemples suivants illustrent l'invention sans en limiter la portée.

## Exemple 1

[[(Indolyl-3)-2 diméthyl-1,1 éthylamino]-3-hydroxy-2 propoxy]-4 éthoxycarbonyl-2 indole. (CM 40 593)

(I) $R_1$ = —$COOC_2H_5$; $R_2$ = H; $R_3$ = —$CH_3$

a) — *(Epoxy-2,3 propoxy)-4 éthoxycarbonyl-2 indole.*

On dissout par chauffage sous atmosphère inerte 4,2 g d'hydroxy-4 éthoxycarbonyl-2 indole et 40 ml d'épichlorhydrine dans 40 ml d'éthanol absolu puis on ajoute 0,82 g de soude en pastilles et porte à reflux pendant 5 heures. On ajoute de l'eau au mélange réactionnel et neutralise par addition d'acide acétique. On évapore les solvants sous vide puis on extrait avec du chlorure de méthylène. On sèche la solution organique sur sulfate de sodium et évapore le solvant à siccité sous vide. On chromatographie sur colonne de gel de silice. Par élution avec le mélange éther-pentane (80—20 volume/volume), on isole le composé attendu (3,5 g), F: 142—144°C.

b) — *CM 40 593.*

On porte au reflux pendant 15 heures le mélange de 3,5 g du composé obtenu ci-dessus et 3 g de (indolyl-3)-2-diméthyl-1,1 éthylamine dans 50 ml d'éthanol. On évapore le solvant sous vide puis on chromatographie le résidu sur une colonne de gel de silice.

En éluant avec le mélange acétate d'éthyle-éthanol (90—10 volume/volume), on obtient le composé attendu (2,8 g). Après recristallisation dans l'acétonitrile, F: 137—142°C.

## Exemples 2 à 14

En opérant comme dans l'exemple 1b) mais en faisant varier les réactifs utilisés, on a isolé les différents produits (I) figurant dans le tableau I ci-après.

## Exemple 15

[[(cyano-5 indolyl-3) diméthyl-1,1 éthylamino]-3 hydroxy-2 propoxy]-4 éthoxycarbonyle-2 indole, fumarate neutre (SR 41831 A)

(I) $R_1$ = —$COOCH_2CH_3$ $R_2$ = (5)—CN $R_3$ = $CH_3$

a) — *(cyano-5 indolyl-3)-2 diméthyl-1,1 éthylamine.*

On chauffe au reflux pendant 5 heures un mélange de 6,45 g de (bromo-5 indolyl-3)-2 diméthyl-1,1 éthylamine et 3,25 g de cyanure cuivreux dans 40 ml de N-méthylpyrrolidone.

On évapore le solvant à 80°C sous vide puis on reprend le résidu dans 50 ml d'une solution d'ammoniaque a 40%; en ajoute du chloroforme et agite pendant 30 minutes. On filtre l'insoluble qu'on lave 5 fois avec 30 ml de chloroforme bouillant. On sépare la phase organique, lave avec de l'eau, sèche sur sulfate de sodium et évapore le solvant à siccité sous vide.

On chromatographie sur une colonne de gel de silice. En éluant avec le mélange acétate d'éthyle-méthanol (80—20 volume/volume) on obtient le produit attendu. On recristallise dans l'éther après décoloration au noir animal; poids: 1,4 g F: 145°C.

b) — *SR 41831 A*

On opère comme dans l'exemple 1b), à partir de la (cyano-5 indolyl-3)-2 diméthyl-1,1 éthylamine obtenue ci-dessus.

Par le même traitement, on obtient le produit attendu sous forme de base huileuse. Cette base est transformée en fumarate neutre par chauffage à l'ébullition avec une quantité stoechiométrique d'acide fumarique au sein de l'éthanol absolu.

Après recristallisation dans l'acétate d'éthyle-éthanol F: 246—248°C.

Les produits de l'invention ont été étudiés en vue de déterminer leur activité pharmacologique et plus spécialement leur activité sur le système cardiovasculaire.

Les produits de l'invention ont été soumis aux épreuves pharmacodynamiques indiquées ci-dessous.

*Action pharmacologique in vivo chez le chien.*

Le chien est anesthésié au pentobarbital sodique, administré par voie intraveineuse à la dose de 30 mg/kg. Une canule placée dans la veine saphène permet les injections intraveineuses des produits. L'animal est intubé et laissé en respiration spontanée.

Le fréquence cardiaque (F.C.) et la pression artérielle systémique (P.A.) et le paramètre

$$\frac{dP}{dt} P^{-1}$$

max qui exprime la contractibilité cardiaque sont enregistrés à l'aide de catéthers et les variations de ces paramètres sont mesurées après injection intraveineuse du produit à tester, chaque produit étant testé à doses croissantes.

*Antagonisme des effets de l'isoprénaline*

L'antagonisme des produits vis-à-vis des effets cardiovasculaires stimulants de l'isoprénaline sur les récepteurs β adrénergiques a été recherché. Les résultats sont présentés dans le tableau II ci-après et exprimés en $DI_{80}$: il s'agit de la dose exprimée en mg/kg qui provoque l'inhibition de 80% de la tachycardie induite par l'isoprénaline administrée par voie intraveineuse.

*Antagonisme des effets de la noradrénaline.*

L'antagonisme des produits vis-à-vis des effets vasculaires provoquès par l'administration intraveineuse de noradrénaline sur les récepteurs α adrénergiques a été recherché. Les résultats présentés dans le tableau II sont exprimés en $DI_{50}$: c'est la dose (mg/kg) qui provoque l'inhibition de 50% de la réponse pressive due à l'administration intraveineuse de noradrénaline.

Après administration intraveineuse, les composés testés présentent donc un profil bêta bloqueur dont l'intensité est plus grande que celle du propanolol pris comme produit de référence. Leur durée d'action est longue (supérieure à 4 heures).

Le même activité se retrouve après administration par voie intraduodénale.

Par ailleurs, les composés testés présentent une activité alphalytique faible ou nulle à la dose β-bloquante.

Les composés selon l'invention montrent également un effet vasodilatateur périphérique exprimé par la diminution de la pression artérielle causée par la diminution des résistances périphériques totales.

Cet effet est confirmé par la technique de la patte perfusée et isolée chez le chien anesthésie par administration intra-artérielle du produit à étudier aux doses variant de 30 à 1000 µg/ml/minute.

Enfin, chez le chien réserpiné et anesthésié, CM 40 593 administré à la dose de 1 mg/kg par voie intraveineuse, montre un léger effet chronotrope positif traduisant un léger effet sympathicomimétique.

Les différents résultats sur le système cardiovasculaire obtenus chez le chien anesthésié ont été confirmés pour certains produits chez le chien vigile après administration du produit par voie orale.

On a pu constater ainsi que les composés de n° de code SR 41719 A, 41716 A, 41779 A, 41780 A et 41828 A, diminuent la fréquence cardiaque ou sont sans action sur celle-ci.

Cette caractéristique opposée à celle de nombreux produits de l'art antérieur est particulièrement favorable dans le traitement de l'hypertension chez l'homme.

En effet l'effet tachycardisant que l'on observe fréquemment chez les produits β-bloquante de cette famille inhibe partiellement l'effet hypotenseur obtenu. Dans le cas des produits selon l'invention l'absence d'effet tachycardisant permet à l'effet hypotenseur de se manifester complètement. Lorsqu'il existe un effet bradycardisant, celui-ci renforce même l'effet hypotenseur du produit.

Enfin les produits selon l'invention sont extrêmement peu toxiques. Ainsi à titre d'exemple le composé CM 40593 présente une dose létale 50 par voie orale comprise entre 3000 et 5000 mg/kg chez la souris mâle et une dose létale 50 supérieure à 5000 mg/kg chez le rat mâle.

Ainsi les produits selon l'invention présentent à la fois des propriétés β-bloquantes, vasodilatatrices et sympathicomimétiques intrinsèques.

Ces produits peuvent donc être utilisés en thérapeutique humaine notamment dans le traitement de l'hypertension, le traitement des arythmies cardiaques et dans le traitement de la maladie coronarienne.

Les produits selon l'invention peuvent être présentés sous les différentes formes destinées à l'administration orale telle que comprimés dosés de 10 à 100 mg ou à l'administration rectale telle que suppositoires dosés de 10 à 100 mg ou sous forme de préparations injectables contenant de 5 à 50 mg de principe actif.

La posologie usuelle est de 2 à 4 comprimés à 20 mg par jour mais exceptionnellement, sous surveillance médicale, elle pourra dépasser notablement ces chiffres.

On indique ci-après quelques exemples de préparation galénique:

Comprimés

| | |
|---|---|
| *CM 40 593* | 20 mg |
| Cellulose microcristalline | 160 mg |
| Lactose | 172 mg |
| stéarate de magnésium | 8 mg |
| | 360 mg |

Suppositoires

| | |
|---|---|
| *CM 40 593* | 40 mg |
| Suppocire C (mélange injectable d'esters d'acide gras naturels) | qsp 3 g |
| Labrafil 2130 C (huile de palme hydrogénée interestérifiée) | |

TABLEAU I

| N° de code | $R_1$ | $R_2$ | $R_3$ | Base en sel | Point de fusion °C (solvant) |
|---|---|---|---|---|---|
| CM 41105 | $-CONH_2$ | H | $CH_3$ | Base | 193—7 (acétate d'éthyle) |
| CM 41165 | $-CH_2OH$ | H | $CH_3$ | Base | 156—8 (chloroforme) |
| SR 41719 A | 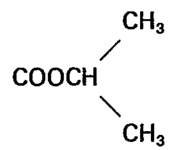 | H | $CH_3$ | chlorohydrate | 144—8 (acétate d'éthyle-éther isopropylique avec 1 molécule d'eau) |
| SR 41828 A | COOH | H | $CH_3$ | chlorhydrate | 244—6 (éthanol aqueux) |
| SR 41370 A | $-C\equiv N$ | H | $CH_3$ | Fumarate neutre | 159—164 (acétate d'éthyle-éthanol) |
| SR 41829 A | 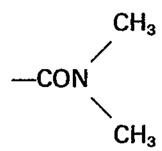 | H | $CH_3$ | Fumarate neutre | 223—6 (acétate d'éthyle-éthanol) |
| SR 41830 A | 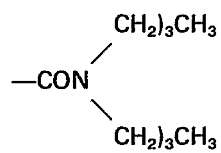 | H | $CH_3$ | Fumarate neutre | 147—9 (acétate d'éthyle-éthanol) |
| SR 41716 A | $COOCH_2CH_3$ | $CH_3(5)$ | $CH_3$ | Fumarate neutre | 252—256 (acétate d'éthyle-éthanol |
| SR 41779 A | $COOCH_2CH_3$ | F(5) | $CH_3$ | Fumarate neutre | 236—239 (acétate d'éthyle-éthanol) |
| SR 41780 A | $COOCH_2CH_3$ | Cl(5) | $CH_3$ | Fumarate neutre | 249—251 (acétate d'éthyle-éthanol) |
| SR 41648 | $COOCH_2CH_3$ | $OCH_3(5)$ | $CH_3$ | Base | 122—124 (chloroforme-éther isopropylique) |
| SR 41832 | $COOCH_2CH_3$ | H | $CH_2OH$ | Base | 120—122°C (acétate d'éthyle) Isomère non déterminé |
| SR 41294 A | $COOCH_2CH_3$ | H | H | Fumarate neutre | 219—221 (éthanol) mélange d'isomères |

TABLEAU II

| N° de code | Effet β-$DI_{80}$ (mg/kg) Isoprènaline | Effet β-$DI_{50}$ mg/kg Noradrénaline | Effets sur les paramètres FC | PA | $\frac{dP}{dt}$ $P^{-1}$max |
|---|---|---|---|---|---|
| CM 40593 | 0,1 | 0,1 | = ou ↗ | ↓ | ↑ |
| CM 41105 | 0,1 | — | ↗ faible | ↓ | ↑ |
| CM 41165 | 0,05 | — | ↘ | ↓ | ↓ |
| SR 41828 A | 0,3 | — | = ou ↘ | ↓ | = |
| SR 41370 A | 0,05 | 0 | ↗ faible | ↓ | ↑ |
| SR 41830 A | 1 | 0 | ↘ | ↗ | ↑ |
| SR 41648 | 0,1 | 0 | ↘ | ↓ | ↓ |
| SR 41832 | 0,1 | 0 | ↘ | ↓ | ↓ |
| SR 41294 A | 0,1 | 0 | ↘ | ↓ | ↓ |
| SR 41831 A | 0,1 | — | ↘ | ↓ | ↓ |

**Revendications pour les Etats Contractants: BE CH DE FR GB IT LI LU NL SE**

1. Composés dérivés de la tryptamine de formule:

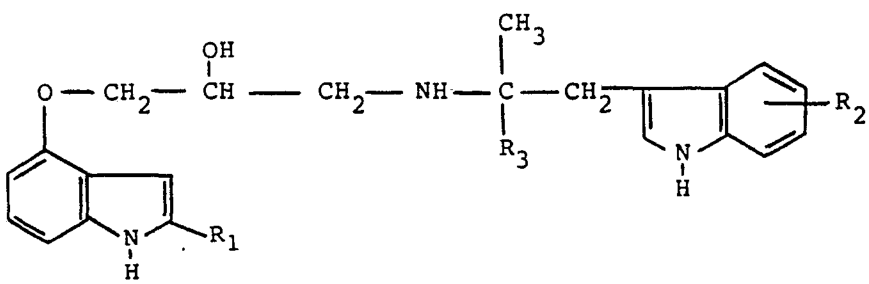

(I)

dans laquelle

$R_1$ représente un groupe $—CH_2OH$, un groupe —C≡N, un groupe

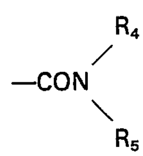

ou encore un groupe $—COOR_6$ dans lequel $R_4$, $R_5$ et $R_6$ désignent un groupe alkyle droit ou ramifié comportant 1 à 4 atomes de carbone ou l'hydrogène,

$R_2$ représente l'hydrogène ou un groupe —C≡N,

$R_3$ désigne l'hydrogène, un groupe $—CH_3$ ou encore un groupe $—CH_2OH$, et les sels d'addition des produits de formule I avec les acides minéraux ou organiques pharmaceutiquement acceptables.

2. Composés dérivés de la tryptamine selon la revendication 1, caractérisés en ce que $R_2$ est l'hydrogène, $R_3$ est le groupe $—CH_3$ et $R_1$ est choisi parmi les groupes ci-après:

$—CONH_2$; $—CH_2OH$; 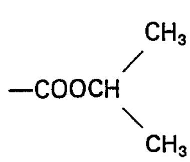 ; —COOH; —C≡N; 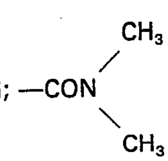 ; 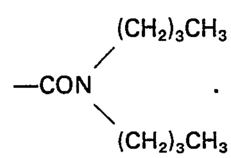 .

3. Composés dérivés de la tryptamine selon la revendication 1, caractérisés en ce que $R_1$ est $—COOCH_2CH_3$, $R_2$ est H et $R_3$ est H ou $—CH_2OH$.

4. Composés dérivés de la tryptamine selon la revendication 1, caractérisés en ce que $R_1$ est $—COOCH_2CH_3$, $R_2$ est H ou (5)—C≡N et $R_3$ est $—CH_3$.

5. Composés dérivés de la tryptamine répondant à la formule:

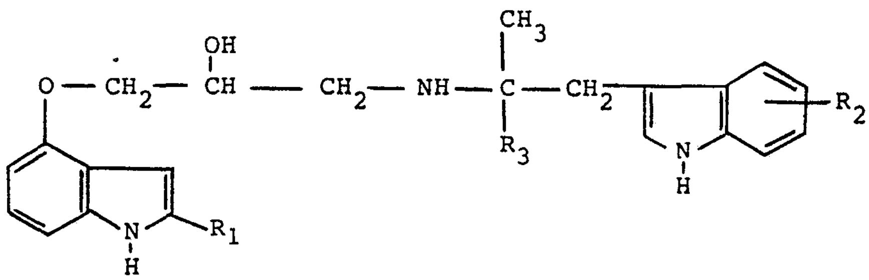

(I)

dans laquelle $R_1$ est le groupe $—COOCH_2CH_3$, $R_3$ est $—CH_3$ et $R_2$ est choisi parmi les groupes ci-après:

(5)—$CH_3$, (5)—F, (5)—Cl, (5)—$OCH_3$.

6. Procédé de préparation des produits de formule I selon l'une quelconque des revendications 1 à 5, caractérisé en ce que l'on fait réagir un époxyde de formule:

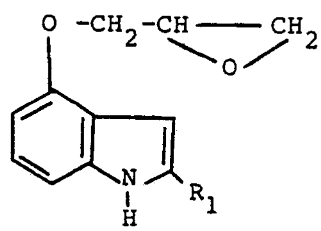

1

sur une amine de formule

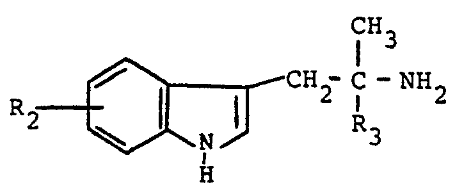

2

la réaction étant réalisée par chauffage de quantités sensiblement équimoléculaires des réactifs éventuellement en solution dans un solvant, les radicaux $R_1$, $R_2$ et $R_3$ étant tels que définis dans l'une quelconque des revendications 1 à 5, et en ce qu'éventuellement on transforme le composé obtenu en l'un de ses sels d'addition avec les acides minéraux ou organiques pharmaceutiquement acceptables.

7. Procédé selon la revendication 6, caractérisé en ce que que la réaction est réalisée en présence de phénol utilisé comme catalyseur.

8. Médicaments utilisables notamment dans le traitement de l'hypertension, des arythmies cardiaques et des maladies coronariennes, caractérisés en ce qu'ils contiennent au moins un produit selon l'une quelconque des revendications 1 à 5.

9. Médicaments selon la revendication 8, caractérisés en ce qu'ils sont conditionnés pour une administration par voie orale ou rectale et contiennent 10 à 100 mg de produit selon la revendication 1.

10. Médicaments selon la revendication 8, caractérisés en ce qu'ils sont conditionnés pour une administration par injection et contiennent de 5 à 50 mg de produit selon l'une quelconque des revendications 1 à 5.

**Revendications pour l'Etat Contractant: AT**

1. Procédé pour l'obtention de composés dérivés de la tryptamine de formule:

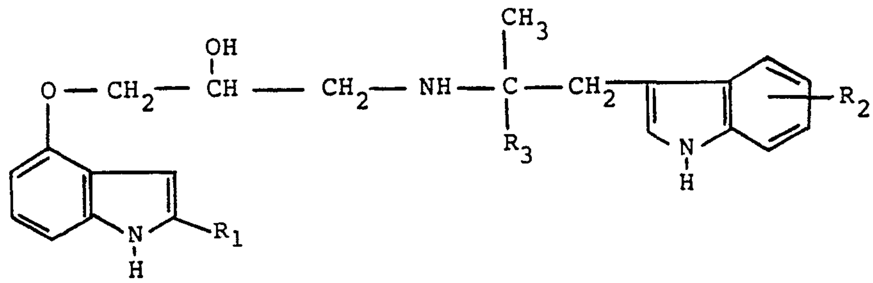

(I)

dans laquelle

$R_1$ représente un groupe $—CH_2OH$, un groupe $—C{\equiv}N$, un groupe

$$—CON\begin{matrix} R_4 \\ R_5 \end{matrix}$$

ou encore un groupe $—COOR_6$ dans lequel $R_4$, $R_5$ et $R_6$ désignent un groupe alkyle droit ou ramifié comportant 1 à 4 atomes de carbone ou l'hydrogène,

$R_2$ représente l'hydrogène ou un groupe $—C{\equiv}N$,

$R_3$ désigne l'hydrogène, un groupe $—CH_3$ ou encore un groupe $—CH_2OH$, et les sels d'addition des produits de formule I avec les acides minéraux ou organiques pharmaceutiquement acceptables, caractérisé en ce que l'on fait réagir un époxyde de formule

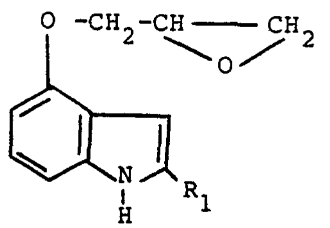

1

sur une amine de formule

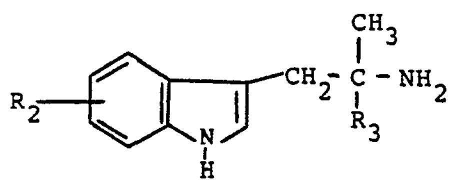

2

la réaction étant réalisée par chauffage de quantités sensiblement équimoléculaires des réactifs éventuellement en solution dans un solvant, les radicaux $R_1$, $R_2$ et $R_3$ étant tels que définis ci-dessus et en ce qu'éventuellement, on transforme le composé obtenu en l'un de ses sels d'addition avec les acides minéraux ou organiques pharmaceutiquement acceptables.

2. Procédé selon la revendication 1, caractérisé en ce que la réaction est réalisée en présence de phénol utilisé comme catalyseur.

3. Procédé selon l'une des revendications 1 ou 2, caractérisé en ce que l'on fait réagir un époxyde de formule *1* dans laquelle $R_1$ est un radical choisi parmi les groupes ci-après:

$$—CONH_2;\ —CH_2OH;\ —COOCH\begin{matrix} CH_3 \\ CH_3 \end{matrix};\ —COOH;\ —C{\equiv}N;\ —CON\begin{matrix} CH_3 \\ CH_3 \end{matrix};\ —CON\begin{matrix} (CH_2)_3CH_3 \\ (CH_2)_3CH_3 \end{matrix}.$$

avec une amine de formule *2* dans laquelle $R_2$ est l'hydrogène et $R_3$ est $—CH_3$.

4. Procédé selon l'une des revendications 1 ou 2, caractérisé en ce que l'on fait réagir un époxyde de formule *1* dans laquelle $R_1$ est $—COOCH_2CH_3$, avec une amine de formule *2* dans laquelle $R_2$ est l'hydrogène et $R_3$ est l'hydrogène ou le groupe $—CH_2OH$.

5. Procédé selon l'une des revendications 1 ou 2, caractérisé en ce que l'on fait réagir un époxyde de

formule *1* dans laquelle $R_1$ est $-COOCH_2CH_3$ avec une amine de formule *2* dans laquelle $R_2$ est H ou (5)—C≡N et $R_3$ est $-CH_3$.

6. Procédé pour l'obtention de composés dérivés de la tryptamine répondant à la formule:

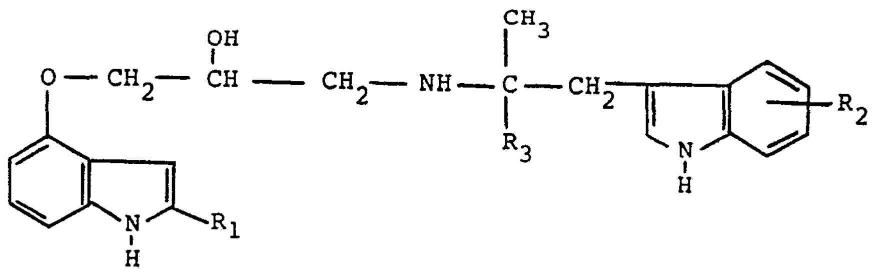

(I)

dans laquelle $R_1$ est le groupe $-COOCH_2CH_3$; $R_3$ est $-CH_3$ et $R_2$ est choisi parmi les groupes ci-après:

(5)—$CH_3$, (5)—F, (5)—Cl, (5)—$OCH_3$.

caractérisé en ce qu'il consiste à faire réagir un époxyde de formule *1* dans laquelle $R_1$ est tel que défini ci-dessus avec une amine de formule *2* dans laquelle $R_2$ et $R_3$ sont tels que définis ci-dessus et à transformer éventuellement ledit composé en l'un de ses sels d'addition d'acides minéraux ou organiques pharmaceutiquement acceptables.

7. Utilisation des composés dérivés de la tryptamine obtenus par le procédé selon l'une quelconque des revendications 1 à 6, pour la préparation de médicaments utilisables notamment pour le traitement de l'hypertension, des arythmies cardiaques et des maladies coronariennes.

**Patentansprüche für die Vertragsstaaten: BE CH DE FR GB IT LI LU NL SE**

1. Von Tryptamin abgeleitete Verbindungen der Formel

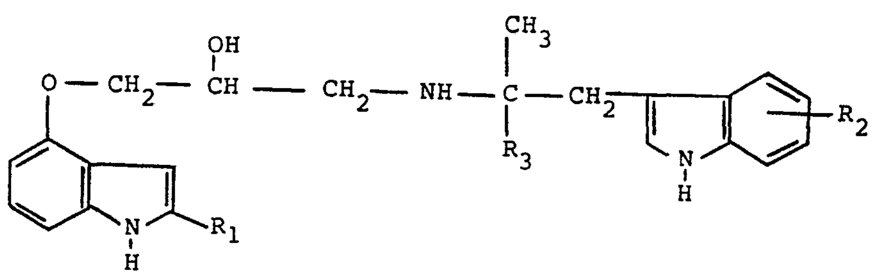

(I)

worin

$R_1$ eine Gruppe $-CH_2OH$, eine Gruppe —C≡N, eine Gruppe

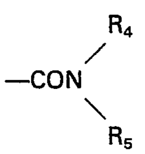

oder aber eine Gruppe $-COOR_6$ darstellt, worin $R_4$, $R_5$ und $R_6$ eine gerade oder verzweigte Alkylgruppe mit 1 bis 4 Kohlenstoffatomen oder Wasserstoff bezeichnen,

$R_2$ für Wasserstoff oder eine Gruppe —C≡N steht,

$R_3$ Wasserstoff, eine Gruppe $-CH_3$ oder aber eine Gruppe $-CH_2OH$ bedeutet, und die Additionssalze der Produkte der Formel I mit pharmazeutisch akzeptablen Mineral- oder organischen Säuren.

2. Von Tryptamin abgeleitete Verbindungen nach Anspruch 1, dadurch gekennzeichnet, daß $R_2$ Wasserstoff ist, $R_3$ für die Gruppe $-CH_3$ steht und $R_1$ ausgewählt ist aus den folgenden Gruppen:

$-CONH_2$; $-CH_2OH$; 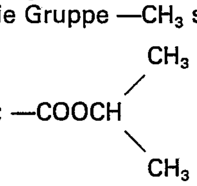 ; —COOH; —C≡N; 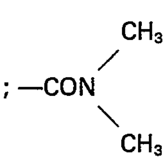 ; 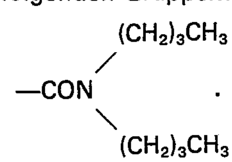 .

3. Von Tryptamin abgeleitete Verbindungen nach Anspruch 1, dadurch gekennzeichnet, daß $R_1$ für $-COOCH_2CH_3$ steht, $R_2$ H bedeutet und $R_3$ H oder $-CH_2OH$ ist.

4. Von Tryptamin abgeleitete Verbindungen nach Anspruch 1, dadurch gekennzeichnet, daß $R_1$ für $—COOCH_2CH_3$ steht, $R_2$ H oder (5)—C≡N darstellt und $R_3$—$CH_3$ ist.

5. Von Tryptamin abgeleitete Verbindungen der Formel

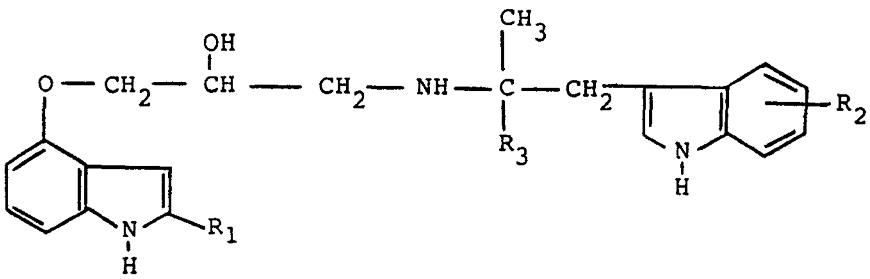

(I)

worin $R_1$ für die Gruppe $—COOCH_2CH_3$ steht, $R_3$ $—CH_3$ bedeutet und $R_2$ ausgewählt ist aus den folgenden Gruppen:

(5)—$CH_3$, (5)—F, (5)—Cl, (5)—$OCH_3$

6. Verfahren zur Herstellung der Produkte der Formel I nach einem der Ansprüche 1 bis 5, dadurch gekennzeichnet, daß man ein Epoxid der Formel

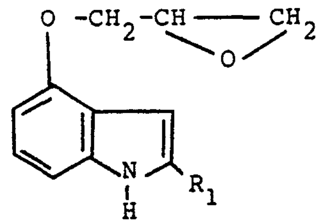

1

mit einem Amin der Formel

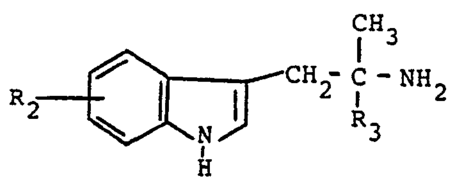

2

reagieren läßt, welche Reaktion durch Erhitzen von im wesentlichen äquimolaren Mengen der Reagenzien, gegebenenfalls in Lösung in einem Lösungsmittel, realisiert wird, wobei die Gruppe $R_1$, $R_2$ und $R_3$ wie in einem der Ansprüche 1 bis 5 definiert sind, und daß man gegebenenfalls die erhaltene Verbindung mit pharmazeutisch akzeptablen Mineral- oder organischen Säuren in eines ihrer Additionssalze überführt.

7. Verfahren nach Anspruch 6, dadurch gekennzeichnet, daß die Reaktion in Gegenwart von Phenol als Katalysator durchgeführt wird.

8. Medikamente, die insbesondere zur Behandlung von Hypertonie, kardialen Arrhythmien und koronaren Erkrankungen geeignet sind, dadurch gekennzeichnet, daß sie mindestens ein Produkt nach einem der Ansprüche 1 bis 5 enthalten.

9. Medikamente nach Anspruch 8, dadurch gekennzeichnet, daß sie für eine Verabreichung auf oralem oder rektalem Weg konditioniert sind und 10 bis 100 mg des Produkts nach Anspruch 1 enthalten.

10. Medikamente nach Anspruch 8, dadurch gekennzeichnet, daß sie für eine Verabreichung durch Injektion konditioniert sind und 5 bis 50 mg des Produkts nach einem der Ansprüche 1 bis 5 enthalten.

**Patentansprüche für den Vertragsstaat: AT**

1. Verfahren zur Herstellung von von Tryptamin abgeleiteten Verbindungen der Formel

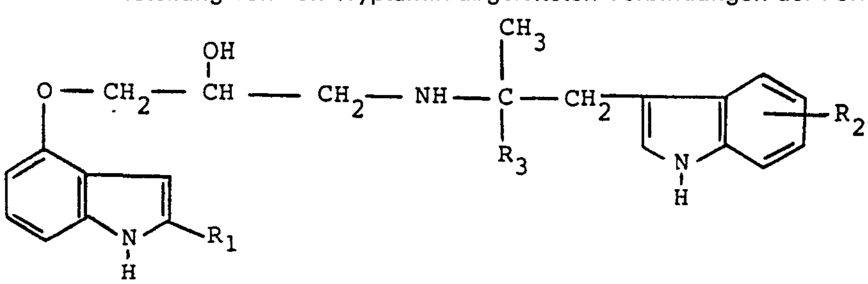

(I)

worin

$R_1$ eine Gruppe $-CH_2OH$, eine Gruppe $-C{\equiv}N$, eine Gruppe

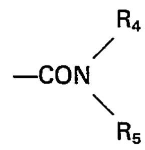

oder aber eine Gruppe $-COOR_6$ darstellt, worin $R_4$, $R_5$ und $R_6$ eine gerade oder verzweigte Alkylgruppe mit 1 bis 4 Kohlenstoffatomen oder Wasserstoff bezeichnen,

$R_2$ für Wasserstoff oder eine Gruppe $-C{\equiv}N$ steht,

$R_3$ Wasserstoff, eine Gruppe $-CH_3$ oder aber eine Gruppe $-CH_2OH$ bedeutet, und der Additionssalze der Produkte der Formel I mit pharmazeutisch akzeptablen Mineral- oder organischen Säuren, dadurch gekennzeichnet, daß man ein Epoxid der Formel

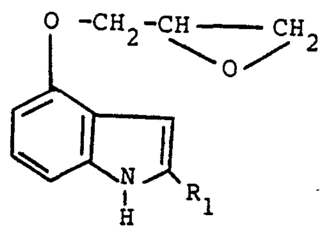

1

mit einem Amin der Formel

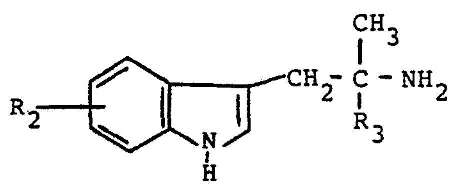

2

reagieren läßt, welche Reaktion durch Erhitzen von im wesentlichen äquimolaren Mengen der Reagenzien, gegebenenfalls in Lösung in einem Lösungsmittel, realisiert wird, wobei die Gruppen $R_1$, $R_2$ und $R_3$ obige Bedeutung haben, und daß man gegebenenfalls die erhaltene Verbindung mit pharmazeutisch akzeptablen Mineral- oder organischen Säuren in eines ihrer Additionssalze überführt.

2. Verfahren nach Anspruch 1, dadurch gekennzeichnet, daß die Reaktion in Gegenwart von Phenol als Katalysator durchgeführt wird.

3. Verfahren nach einem der Anspruch 1 oder 2, dadurch gekennzeichnet, daß man ein Epoxid der Formel *1*, worin $R_1$ ausgewählt ist aus den folgenden Gruppen:

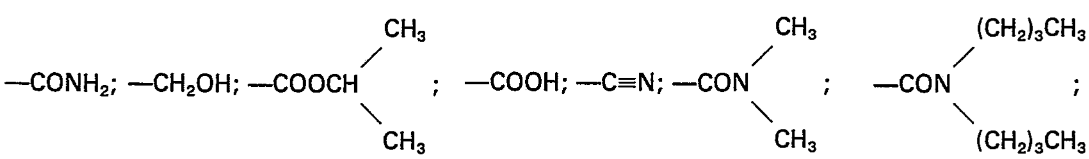

mit einem Amin der Formel *2*, worin $R_2$ Wasserstoff ist und $R_3$ für $-CH_3$ steht, reagieren läßt.

4. Verfahren nach einem der Ansprüche 1 oder 2, dadurch gekennzeichnet, daß man ein Epoxid der Formel *1*, worin $R_1$ $-COOCH_2CH_3$ ist, mit einem Amin der Formel *2*, worin $R_2$ für Wasserstoff steht und $R_3$ Wasserstoff oder die Gruppe $-CH_2OH$ ist, reagieren läßt.

5. Verfahren nach einem der Ansprüche 1 oder 2, dadurch gekennzeichnet, daß man ein Epoxid der Formel *1*, worin $R_1$ für $-COOCH_2CH_3$ steht, mit einem Amin der Formel *2*, worin $R_2$ H oder (5)$-C{\equiv}N$ bedeutet und $R_3$ $-CH_3$ ist, reagieren läßt.

6. Verfahren zur Herstellung von von Tryptamin abgeleiteten Verbindungen der Formel

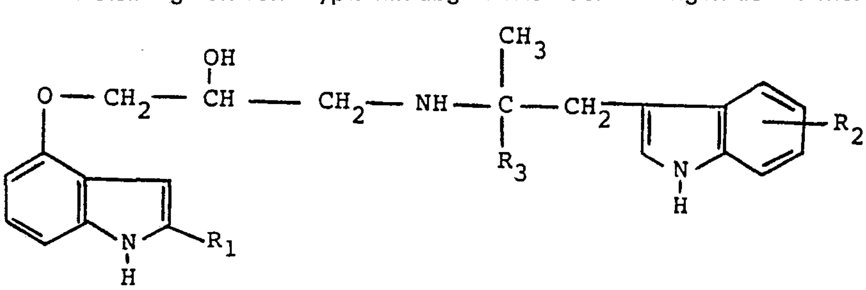

(I)

worin $R_1$ für die Gruppe $-COOCH_2CH_3$ steht; $R_3$ $-CH_3$ bedeutet und $R_2$ ausgewählt ist aus den folgenden Gruppen:

(5)—$CH_3$, (5)—F, (5)—Cl, (5)—$OCH_3$

dadurch gekennzeichnet, daß es darin besteht, daß man ein Epoxid der Formel *1,* worin $R_1$ obige Bedeutung hat, mit einem Amin der Formel *2,* worin $R_2$ und $R_3$ obige Bedeutung haben, reagieren läßt und gegebenenfalls diese Verbindung in eines ihrer Additionssalze von pharmazeutisch akzeptablen Mineral- oder organischen Säure überführt.

7. Verwendung der durch das Verfahren nach einem der Ansprüche 1 bis 6 erhaltenen, von Tryptamin abgeleiteten Verbindungen zur Herstellung von Medikamenten, die insbesondere zur Behandlung von Hypertonie, kardialen Arrhythmien und koronaren Erkrankungen geeignet sind.

**Claims for the Contracting States: BE CH DE FR GB IT LI LU NL SE**

1. Compounds derived from tryptamine of formula:

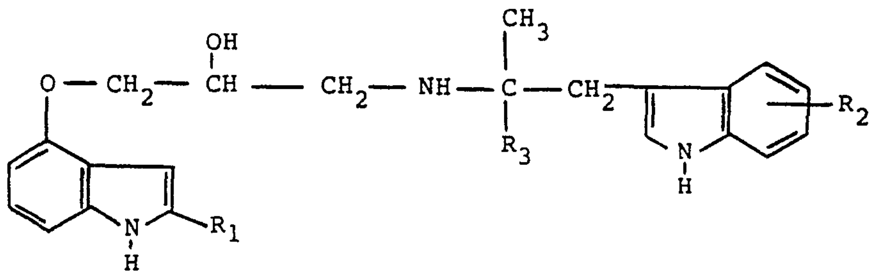

(I)

in which

$R_1$ represents a $-CH_2OH$ group, a $-C\equiv N$ group, a

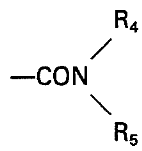

group, or still a $-COOR_6$ group in which $R_4$, $R_5$ and $R_6$ designate a straight or branched alkyl group having 1 to 4 atoms of carbon or hydrogen,

$R_2$ represents hydrogen or $-C\equiv N$ group,

$R_3$ designates hydrogen, a $-CH_3$ group or still a $-CH_2OH$ group, and the addition salts of the products of formula I with the pharmaceutically acceptable mineral or organic acids.

2. Compounds derived from tryptamine according to formula 1, characterized in that $R_2$ is hydrogen, $R_3$ is the $-CH_3$ group and $R_1$ is selected from the following groups:

$-CONH_2$; $-CH_2OH$; 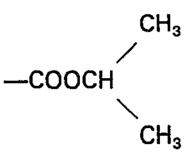 ; $-COOH$; $-C\equiv N$; 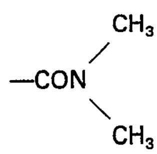 ; 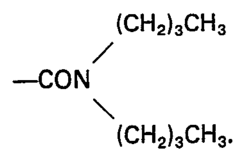

3. Compounds derived from tryptamine according to claim 1, characterized in that $R_1$ is $-COOCH_2CH_3$, $R_2$ is H and $R_3$ is H or $-CH_2OH$.

4. Compounds derived from tryptamine according to claim 1, characterized in that $R_1$ is $-COOCH_2CH_3$, $R_2$ is H or (5)—$C\equiv N$ and $R_3$ is $-CH_3$.

5. Compounds derived from tryptamine responding to the formula:

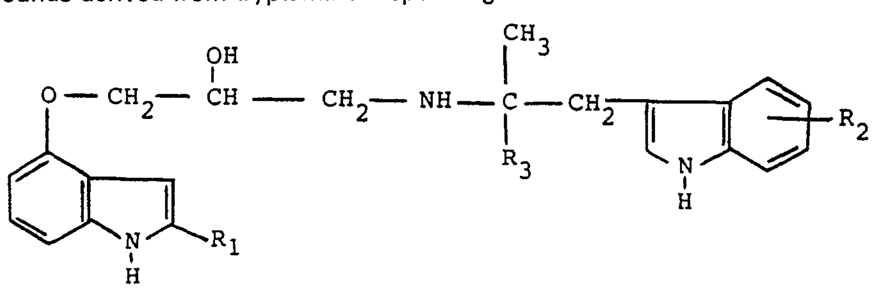

(I)

in which $R_1$ is the $-COOCH_2CH_3$ group, $R_3$ is $-CH_3$ and $R_2$ is selected from the following groups:

(5)$-CH_3$, (5)$-F$, (5)$-Cl$, (5)$-OCH_3$.

6. Process for preparing products of formula I according to any one of claims 1 to 5, characterized in that an epoxy of formula:

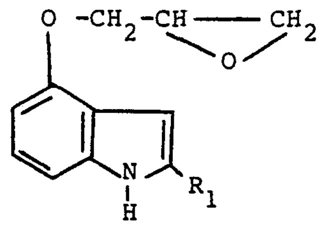

1

is reacted on an amine of formula

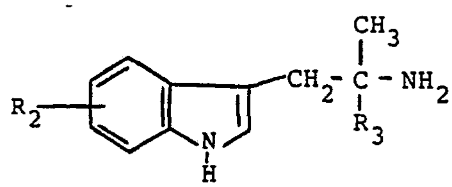

2

the reaction being carried out by heating substantially equimolecular quantities of reagents optionally in solution in a solvent, the $R_1$, $R_2$ and $R_3$ radicals being such as defined in any one of claims 1 to 5, and in that the compound thus obtained is optionally converted into one of its addition salts with the pharmaceutically acceptable mineral or organic acids.

7. Process according to claim 6, characterized in that the reaction is carried out in the presence of phenol used as catalyst.

8. Drugs usable in particular in the treatment of hypertension, of cardiac arhythmia and coronary disorders, characterized in that they contain at least one product according to any one of claims 1 to 5.

9. Drugs according to claim 8, characterized in that they are packed for an oral or rectal administration and contain 10 to 100 mg of the product according to claim 1.

10. Drugs according to claim 8, characterized in that they are packed for an administration by injection and contain 5 to 50 mg of the product according to any one of claims 1 to 5.

**Claims for the Contracting State: AT**

1. Process for obtaining compounds derived from tryptamine of formula:

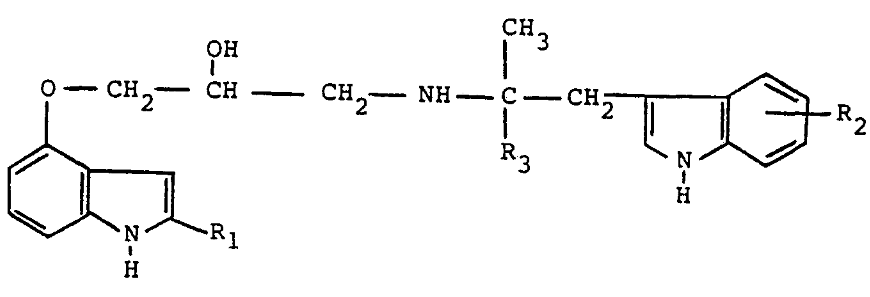

(I)

in which

$R_1$ represents a $-CH_2OH$ group, a $-C{\equiv}N$ group, a

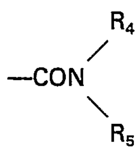

group, or still a $-COOR_6$ group in which $R_4$, $R_5$ and $R_6$ designate a straight or branched alkyl group having 1 to 4 atoms of carbon or hydrogen,

$R_2$ represents hydrogen or $-C{\equiv}N$ group,

$R_3$ designates hydrogen, a $-CH_3$ group or still a $-CH_2OH$ group, and the addition salts of the products

of formula I with the pharmaceutically acceptable mineral or organic acids, characterized in that an epoxy of formula

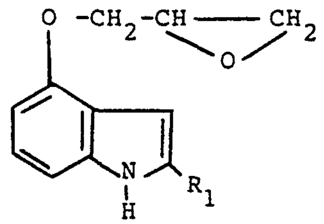

1

is reacted on an amine of formula

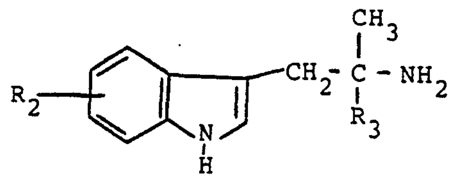

2

the reaction being carried out by heating substantially equimolecular quantities of reagents optionally in solution in a solvent, the radicals $R_1$, $R_2$ and $R_3$ being such as defined above and in that optionally, the compound thus obtained is converted into one of its addition salts with pharmaceutically acceptable mineral or organic acids.

2. Process according to claim 1, characterized in that the reaction is carried out in the presence of phenol used as catalyst.

3. Process according to any one of claims 1 or 2, characterized in that an epoxy of formula *1* in which $R_1$ is a radical selected from the following groups:

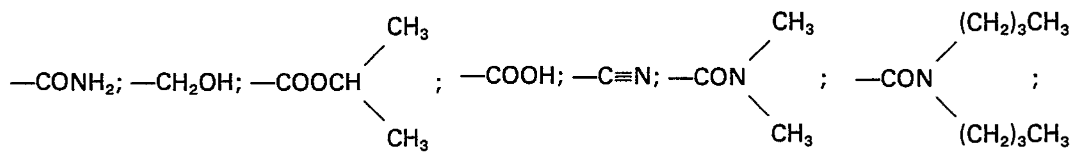

is reacted with an amine of formula *2* in which $R_2$ is hydrogen and $R_3$ is $—CH_3$.

4. Process according to any one of claims 1 or 2, characterized in that an epoxy of formula *1* in which $R_1$ is $—COOCH_2CH_3$ is reacted with an amine of formula *2* in which $R_2$ is hydrogen and $R_3$ is hydrogen or the $—CH_2OH$ group.

5. Process according to any one of claims 1 or 2, characterized in that an epoxy of formula *1* in which $R_1$ is $—COOCH_2CH_3$ is reacted with an amine of formula *2* in which $R_2$ is H or (5)—C≡N and $R_3$ is $—CH_3$.

6. Process for obtaining compounds derived from tryptamine responding to the formula:

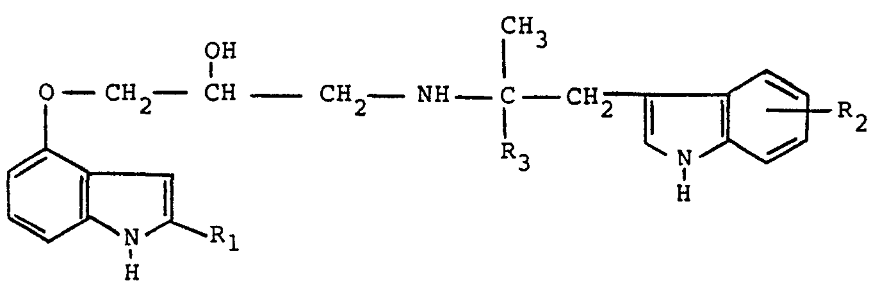

(I)

in which $R_1$ is the $—COOCH_2CH_3$ group; $R_3$ is $—CH_3$ and $R_2$ is selected from the following groups:

(5)—$CH_3$, (5)—F, (5)—Cl, (5)—$OCH_3$

characterized in that it consists in reacting an epoxy of formula *1* in which $R_1$ is such as defined above with an amine of formula *2* in which $R_2$ and $R_3$ are such as defined above and in optionally converting said compound into one of its addition salts of pharmaceutically acceptable mineral or organic acids.

7. Use of compounds derived from tryptamine obtained by the process according to any one of claims 1 to 6, for the preparation of drugs usable in particular in the treatment of hypertension, cardiac arhythmia and coronary disorders.